Europäisches Patentamt

European Patent Office

Office européen des brevets

(19)

(11) Publication number: **0 182 984 B1**

# EUROPEAN PATENT SPECIFICATION

(12)

(45) Date of publication of patent specification: **04.11.92** (51) Int. Cl.5: **A61K 37/02**, C07K 7/10

(21) Application number: **85110985.0**

(22) Date of filing: **30.08.85**

(54) Use of fully synthetic alpha-human atrial natriuretic peptide (alpha-hANap).

(30) Priority: **28.11.84 DE 3443257**

(43) Date of publication of application:
**04.06.86 Bulletin 86/23**

(45) Publication of the grant of the patent:
**04.11.92 Bulletin 92/45**

(84) Designated Contracting States:
**AT BE CH DE FR GB IT LI LU NL SE**

(56) References cited:
**EP-A- 0 116 784
EP-A- 0 147 193
EP-A- 0 159 943
WO-A-85/04870**

**BIOCHEMICAL AND BIOPHISICAL RESEARCH
COMMUNICATIONS, volume 118, no. 1, 13th
January 1984, pages 131-139 NEW YORK (US)
Kenji KANGAWA et al.: "Purification and
complete amino acid sequence of alpha-
human atrial natriuretic polypeptide
(alpha-hANP)"**

**EUROPEAN JOURNAL OF PHARMACOLOGY,
volume 103, nos. 3/4, 17th August 1984,
pages 363-366, Elsevier Science Publishers
AMSTERDAM (NL) Tokihito YUKUMURA et al.:**

**"Renal effects of a synthetic alpha-human
atrial natriuretic polypeptide (alpha-hANP) in
anesthetized dogs"**

(73) Proprietor: **PHARMA BISSENDORF PEPTIDE
GMBH
Karl-Wiechert-Allee 3
W-3000 Hannover 61(DE)**

(72) Inventor: **Hesch, Rolf-Dieter, Prof. Dr.
Gartenweg 12
W-3004 Isernhagen(DE)**

(74) Representative: **Werner, Hans-Karsten, Dr. et
al
Deichmannhaus am Hauptbahnhof
W-5000 Köln 1(DE)**

EP 0 182 984 B1

**Description**

BACKGROUND OF THE INVENTION

A number of medicaments are known for treating ascites in chronic liver diseases, vasopressin disregulation, posterior pituitary malfunction, the psychotropic effects induced thereby, and hypertension caused by a pheocromocyton of the kidney. However, in most cases these prior art medicaments only indirectly act onto the symptoms of the disease and, in addition, cause more or less serious side-effects to occur. They, at least, burden the metabolism when they are being decomposed.

In recent years, atrial extracts from rat atria have been thoroughly investigated. In the course of these studies, various peptides have been isolated that show more or less strong natriuretic and diuretic effects, and in addition, can relax the smooth muscle of vessels. The nomenclature of these peptides has so far been rather confusing (atrial natriuretic factor, Auriculin A, Cardionatrin, Auriculin B, Atriopeptin I, II and III, gamma- and alpha-atrial natriuretic factor). It is believed that this variety of names was in part caused by preparative artefacts and because different peptides were prepared from a common precursor. For the human peptides (as well as for the rat peptides), a common precursor could now be prepared from cloned cDNA that had been prepared from atrial mRNA. The human pre-peptide comprises 151 amino acids. The first 25 amino acids of this peptide conform to the pre-peptide proportion designated as the signal peptide which controls processing along the ribosomal synthesis and the subsequent secretion. Thereafter, a peptide comprising 126 amino acids is released which is the gamma-hANaP having a molecular weight of exactly 13,000 and which has also been identified in human atria.

It has now been found that the essential biologically active peptide corresponds to the last 28 amino acids at the C-terminal end of gamma-hANaP. This peptide has been given the designation of alpha-human atrial natriuretic peptide (alpha-hANaP). The sequence of the amino acids has been elucidated and published by Misono et al., Biochem. Biophys. Res. Commun. 119, (2) 524-529 (1984).

Kanagawa and Matsuo describe diuretic and natriuretic activities as well as vasorelaxant activities and chicken rectum relaxant activities of alpha-hANaP, prepared from human atrial tissue (Biochem. Biophys. Res. Commun. 118, 131-139 (1984).

In reference Eur. J. Pharm. 103, 363-366 (1984), Matsuo et al. investigate the effects of a synthetic alpha-hANaP synthesized by solid phase procedures conducted on a chloromethylated polystyrene resin, on renal hemodynamics and urine formation in anesthesized dogs.

The intermediate document EP-A-0 147 193 of the above mentioned inventors discloses a process for the production of synthetic alpha-hANaP by Merrifield's method.

It is the object of the present invention to provide a medicament for the treatment of cardiac insufficiency by the improvement of renal functions diuresis, sodium excretion reduction of oedematosis and lowering of the blood pressure which also effects improvement of pituitary malfunction which is involved in the pathologic state concerned as well as new use for the treatment of the above-identified diseases that (1) will directly participate in the disease mechanism, (2) that are non-toxic, and (3) will not burden the metabolism.

This is solved by the use of a fully synthetic alpha-human atrial natriuretic peptide (alpha-hANaP) for the preparation of a medicament for treating ascites in chronic liver diseases, vasopressin disregulation, posterior pituitary malfunction, the psychotropic effects induced thereby, and hypertension caused by a pheocromocyton of the kidney. These effects are already observed at a peptide concentration level of $10^{-9}$. However, due to the low toxicity and good compatibilty of the peptide to and with the body, higher concentrations of the peptide also may be injected or infused.

The synthesis of alpha-hANaP follows the solid state synthesis method by Merrifield (1963) involving the use of benzhydrylamine as the supporting polymer in an automatic peptide synthesizer. See Merrifield, R.B., "Solid phase peptide synthesis. I. The synthesis of a tetrapeptide", JACS 85, 2149-2156 (1963). The crude preparation is pre-purified on Sephadex-G 25 (F) and after-purified by means of semi-preparative high pressure liquid chromatography (HPLC). Thus, the fully synthetic replicate of the last C-terminal amino acids (124 through 151) of gamma-hANaP is formed. The presence of the disulfide linkage between the amino acids 7 and 23 appears to be essential for biological activity. The product has a molecular weight of 3,082.6 and is obtained in a freeze-dried form as a white and flaky product having a low specific density. The purity of the product is at least 99%. It may be packaged in amounts of from 10 to 500 $\mu$g and dissolved in physiological saline (0.9% of NaCl) prior to use.

Examination of the acute toxicology in animal tests has shown that, at a dosage level of 100 $\mu$g/kg of body weight, no toxic reactions of the conventional biological control parameters (biochemistry) occur. An $LD_{50}$ could so far not be determined. No serious side-effects to the heart and circulation system, to the bronchial system, to the liver and renal functions, to the gonads, and to the central nervous system were observed.

The pharmacological activity in the treatment of the aforementioned diseases ensues from the observed natriuretic effect which, upon intravenous administration, results in a rapid increase of diuresis and sodium excretion. It has further been observed that the fully synthetic alpha-hANaP is capable of reversing the vasoconstrictive effect of noradrenaline. Further it has been observed that the substance inhibits the vasoconstrictive effect of angiotensin. Of particular interest are those cases of serious heart insufficiency with generalized oedematosis that do not respond to conventional therapy. The action of the substance onto the blood pressure-controlling systems and the vasopressin systems are also of a significant importance, so that posterior pituitary malfunctions and psychotropic effects caused thereby are indicated. It cannot be excluded that a more intensive clinical examination of the instant substance will result in the finding of further interesting indications.

Thus, the present invention relates to the use of a fully synthetic alpha-human atrial natriuretic peptide (alpha-hANaP) for the preparation of a medicament for treating ascites in chronic liver diseases, vasopressin disregulation, posterior pituitary malfunction, the psychotropic effects induced thereby, and hypertension caused by a pheocromocyton of the kidney in a highly purified injectable or infusible form.

The highly purified fully synthetic alpha-human atrial natriuretic peptide (alpha-hANaP) also is believed to be highly active in treating patients suffering from hypertension caused by a pheocromocyton of the kidney. To date this affliction is treated with peripheric ganglion blockers such as phenoxybenzamin. Severe side reactions are caused requiring the patient to be bedridden. Treatment of this form of hypertension with calcium channel blockers such as Nifidepin met with little success.

Use of the highly purified fully synthetic alpha-human atrial natriuretic peptide (alpha-hANaP) is expected to cause little or no side effects since the active compound appears to block the action of catecholamines.

To date the production of the fully synthetic highly purified alpha-hANaP has been carried out by the firm Bachem Inc. Fine Chemicals at the direction of the present inventor in accordance with the Merrifield solid phase synthesis method using benzhydrylamine resin as the supporting polymer in an automatic peptide synthesizer.

Each amino acid residue was attached by a method involving the use of trifluoroacetic acid washes to remove excess acid, an organic base to neutralize the system, washing to remove formed salts, dicyclohexylcarbodiimide to couple the next amino acid, and washing to remove excess reagents and by-products.

The crude preparation was pre-purified on Sephadex-G 25 (F) and after-purified by means of semi-preparative high pressure liquid chromatography (HPLC). More particularly, cleavage and concomitant deprotection of the peptides were achieved using HF in the presence of anisole. After HF was removed and thorough vacuum drying had taken place, the resin was washed with ether. The dried resin was immediately extracted with 2N acetic acid and the extracts were freeze-dried and purified by gel chromatography on Sephadex-G25(F) with a mixture of 25 % acetic acid and 0.001 % $\beta$-mercaptoethanol as eluent. The after-purification was a semi-preparative reverse phase HPLC technique on Bondapak $C^{18}$ with a gradient of acetonitrile in 0.25 N triethylammonium phosphate (HPLC) using a 0.1 percent trifluoroacetic acid and acetonitrile gradient.

The identity has been confirmed by amino acid analysis. The purity and homogeneity and the absence of pyrogen were checked. The amino acid sequence of alpha-hANaP is as follows:

$H_2N$-Ser-Leu-Arg-Arg-Ser-Ser-Cys-Phe-Gly-Gly-Arg-Met-Asp-Arg

└ S-S ┐

Ile-Gly-Ala-Gln-Ser-Gly-Leu-Gly-Cys-Asn-Ser-Phe-Arg-Tyr-COOH

Molecular weight: 3,082.6
A check by means of HPLC confirmed that the purity was in excess of 99%.

Example

3

Fully synthetic highly purified alpha-hANaP was apportioned into amounts of 50 $\mu$g of a lyophilized powder without excipient into 2 ml glass vials. To effect dissolution, there was used 1 ml of a physiological saline (0.9% NaCl). These solutions can be readily injected or infused. Basically, there is also the possibility to prepare the peptide in physiological saline or a different isotonic physiologically compatible solution and to store this preparation. If desired, a physiologically acceptable disinfectant and/or stabilizer may be added to these solutions.

**Claims**

1. The use of a fully synthetic alpha-human atrial natriuretic peptide (alpha-hANaP) for the preparation of a medicament for treating ascites in chronic liver diseases, vasopressin disregulation, posterior pituitary malfunction, the psychotropic effects induced thereby, and hypertension caused by a pheocromocyton of the kidney.

2. The use according to claim 1 whereby the peptide is in an injectible or infusible form.

3. The use according to claims 1 and 2 whereby said peptide is at least 98% pure.

4. The use according to claims 1 to 3 whereby said peptide contains the following amino acid sequence:

$$H_2N-Ser-Leu-Arg-Arg-Ser-Ser-Cys-Phe-Gly-Gly-Arg-Met-Asp-Arg$$
$$| $$
$$S-S$$
$$|$$
$$Ile-Gly-Ala-Gln-Ser-Gly-Leu-Gly-Cys-Asn-Ser-Phe-Arg-Tyr-COOH ,$$

**Patentansprüche**

1. Verwendung eines vollsynthetischen alpha-humanen atrialen natriuretischen Peptids ($\alpha$-hANaP) zur Herstellung eines Medikaments zur Behandlung von Aszites bei chronischen Lebererkrankungen, Vasopressin-Disregulation, Funktionsstörungen des Hypophysenhinterlappens, den hierdurch bedingten psychotropen Effekten sowie durch Nieren-Phäochromozytom hervorgerufene Hypertonie.

2. Verwendung nach Anspruch 1, wobei das Peptid in injizierbarer oder infusionierbarer Form vorliegt.

3. Verwendung nach Anspruch 1 und 2, wobei das Peptid wenigstens 98% rein ist.

4. Verfahren nach den Ansprüchen 1 bis 3, wobei das Peptid folgende Aminosäure-Sequenz enthält:

$$H_2N-Ser-Leu-Arg-Arg-Ser-Ser-Cys-Phe-Gly-Gly-Arg-Met-Asp-Arg$$
$$|$$
$$S——S$$
$$|$$
$$Ile-Gly-Ala-Gln-Ser-Gly-Leu-Gly-Cys-Asn-Ser-Phe-Arg-Tyr-COOH.$$

**Revendications**

1. L'utilisation d'un alpha-peptide natriurétique auriculaire humain (alpha-hANaP) entièrement synthétique pour la préparation d'un médicament destiné au traitement de l'ascite dans des affections chroniques du foie, d'une mauvaise régulation de la vasopressine, d'un dérèglement antéhypophysaire, des effets psychotropes qui en résultent, et de l'hypertension provoquée par un phéochromocytome du rein.

**2.** L'utilisation selon la revendication 1, dans laquelle le peptide est sous une forme administrable par injection ou perfusion.

**3.** L'utilisation selon les revendications 1 et 2, dans laquelle ledit peptide a une pureté d'au moins 98 %.

**4.** L'utilisation selon les revendications 1 à 3, dans laquelle ledit peptide contient la séquence d'acides aminés suivante :

```
H2N-Ser-Leu-Arg-Arg-Ser-Ser-Cys-Phe-Gly-Gly-Arg-Met-Asp-Arg
                              |
                             S-S
                                 |
Ile-Gly-Ala-Gln-Ser-Gly-Leu-Gly-Cys-Asn-Ser-Phe-Arg-Tyr-COOH .
```